# EUROPEAN PATENT SPECIFICATION

(11) **EP 0 815 790 B1**
(45) Date of publication and mention of the grant of the patent: **15.11.2000**
(21) Application number: 96110684.6
(22) Date of filing: 02.07.1996
(51) Int. Cl.: A61B 5/0225

(54) **Blood pressure monitor**
Blutdruckmesser
Appareil de mesure de la pression sanguine

(43) Date of publication of application: 07.01.1998
(73) Proprietor: COLIN CORPORATION, Komaki-shi Aichi 485 (JP)
(72) Inventor: Yokozeki, Akihiro, c/o Colin Corporation, Komaki-shi, Aichi-ken (JP); Narimatsu, Kiyoyuki, c/o Colin Corporation, Komaki-shi, Aichi-ken (JP)
(74) Representative: Winter, Brandl, Fürniss, Hübner, Röss, Kaiser, Polte Partnerschaft

(56) References cited:
- EP-A- 0 655 219
- EP-A- 0 696 433
- EP-A- 0 698 370
- US-A- 5 000 187

## Description

### BACKGROUND OF THE INVENTION

### Field of the Invention

The present invention relates to a blood pressure monitor including an inflatable cuff.

### Related Art Statement

There is known a blood pressure (BP) monitor which includes an inflatable cuff adapted to be wound around a body portion, e.g., upper arm, of a living subject, e.g., patient, to press the body portion. The BP monitor functions as an automatic BP measuring device which periodically measures a BP value of the subject by increasing the cuff pressure and thereby pressing the body portion of the subject. However, if the period or interval of the BP measurements effected by the BP monitor is shortened for improving the accuracy of monitoring of subject's blood pressure, the frequency of pressing of subject's body portion is increased, which causes the subject to feel discomfort.

In the above-indicated background, it has been proposed to increase the pressure of an inflatable cuff being wound around a body portion of a living subject, up to a predetermined value, detect a pulse wave that is a pressure oscillation produced in the cuff, and estimate a BP value of the subject based on the magnitude of the pulse wave. This technique is disclosed in, e.g., Japanese Patent Application laid open for inspection purposes under Publication No. 61(1986)-103432, or Japanese Patent Application laid open for inspection purposes under Publication No. 60(1985)-241422.

A blood pressure monitor according to the first part of claim 1 is known from U.S. Patent No. 5 000 187.

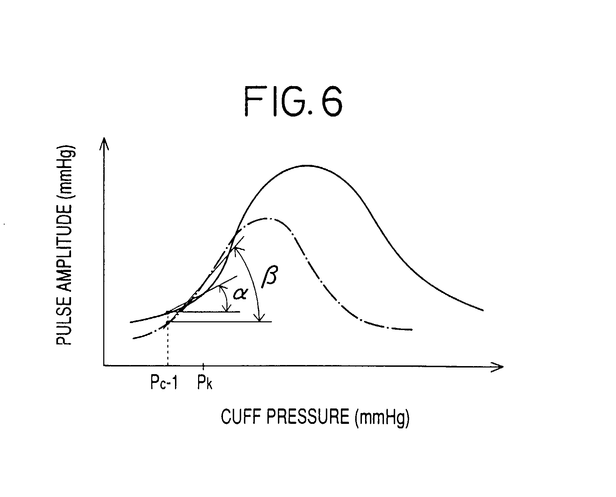

Regarding the above-indicated conventional BP monitor techniques, however, there are known some cases where it is difficult to detect a change of magnitudes of pulse waves which well reflects a change of blood pressure of a living subject, if BP values are estimated based on the pulse waves detected at a considerably low cuff pressure, which contributes to reducing the discomfort felt by the subject. More specifically described, respective amplitudes of pulses of a pulse wave which is detected from an inflatable cuff being wound around a body portion of a living subject whose blood pressure is normal, has an envelope indicated at solid line in the graph of Fig. 6. In contrast, amplitudes of pulses of a pulse wave obtained from a living subject whose blood pressure is low, has an envelope indicated at one-dot chain line in Fig. 6. In the case where amplitudes of pulses of a pulse wave are detected at a considerably low cuff pressure, e.g., pressure, P_{K}, in Fig. 6, an amount of change of the pulse amplitudes with respect to an amount of change of blood pressure of a living subject may be too small. Thus, when the BP monitor is used at the low cuff pressure P_{K}, it may not be able to monitor the blood pressure of the subject with high accuracy.

### SUMMARY OF THE INVENTION

It is therefore an object of the present invention to provide a blood pressure monitor which includes an inflatable cuff and which monitors with high accuracy the diastolic blood pressure of a living subject without causing the subject to feel discomfort.

The above object has been achieved according to the present invention, which provides a blood pressure monitor including an inflatable cuff which is adapted to be wound around a body portion of a living subject to press the body portion, a pressure sensor which detects a pressure in the cuff, a cuff-pressure regulating device which increases the pressure of the cuff, pulse-amplitude determining means for determining an amplitude of each of pulses of a pulse wave which are produced in the cuff and detected by the pressure sensor while the pressure of the cuff is increased by the cuff-pressure regulating device, candidate determining means for determining, as a diastolic blood pressure candidate, a pressure of the cuff which is detected by the pressure sensor and which corresponds to an amplitude of a first pulse of the pulses determined by the pulse-amplitude determining means, by judging whether the amplitude of the first pulse is not greater than a reference value which is smaller than an amplitude of at least one second pulse of the pulses, by a predetermined proportion of the amplitude of the second pulse, the amplitude of the second pulse being determined by the pulse-amplitude determining means after the amplitude of the first pulse is determined, and blood-pressure determining means for determining, as a monitor diastolic blood pressure value, the pressure of the cuff corresponding to the amplitude of the first pulse, when the candidate determining means determines, as the diastolic blood pressure candidate, the pressure of the cuff corresponding to the amplitude of the first pulse, with respect to a predetermined number of the at least one second pulse.

In the blood pressure (BP) monitor constructed as described above, the predetermined number may be one, two, or a greater number. For example, the predetermined number is three. Thus, the present BP monitor may determine a monitor diastolic BP value of a living subject at a pressure level which is higher than the diastolic BP value and which corresponds to the "third" one of the second pulses determined after the first pulse. The thus determined monitor diastolic BP value enjoys high accuracy. In addition, since the pressure level where the monitor diastolic BP value is determined is considerably low, the subject does not feel discomfort.

According to a preferred feature of the present invention, the candidate determining means comprises judging means for judging whether the amplitude of the first pulse is not greater than a reference value which is smaller than an amplitude of each of a plurality of second pulses of the pulses, by a predetermined proportion of the amplitude of the each second pulse, the respective amplitudes of the second pulses being determined by the pulse-amplitude determining means after the amplitude of the first pulse is determined.

According to another preferred feature of the present invention, the cuff-pressure regulating device comprises pressure increasing means for stepwise increasing the pressure of the cuff by alternately increasing the cuff pressure and maintaining the cuff pressure at each of a plurality of different pressure values, and the pulse-amplitude determining means determines an amplitude of at least one pulse which is produced in the cuff and detected by the pressure sensor while the cuff pressure is maintained at the each pressure value. The pressure increasing means may increase the cuff pressure by a constant pressure increase amount, for each time or step, or may increase the cuff pressure by an increase amount which is variable depending upon the current cuff pressure. The pulse-amplitude determining means may determine an amplitude of a single pulse detected by the pressure sensor while the cuff pressure is maintained at each pressure value, or an average of respective amplitudes of two or more pulses detected while the cuff pressure is maintained at each pressure value. The thus determined pulse amplitude or amplitudes enjoy high accuracy because they are free from adverse influences resulting from the increasing of the cuff pressure. Therefore, the monitor diastolic BP values of the subject are determined with accuracy based on the pulse amplitudes.

According to another preferred feature of the present invention, the blood-pressure determining means comprises monitor means for iteratively determining the monitor diastolic blood pressure value. The monitor means may periodically determine the monitor diastolic blood pressure value at a predetermined period or interval of time (i.e., monitor cycle time).

According to another preferred feature of the present invention, the BP monitor further comprises abnormality identifying means for identifying an abnormality of the monitor diastolic blood pressure values iteratively determined by the monitor means.

According to another preferred feature of the present invention, the abnormality identifying means comprises means for identifying the abnormality based on at least one of an amount of change of a last determined value of the monitor diastolic blood pressure values from an average of the monitor diastolic blood pressure values, and a rate of change of the last determined value of the monitor diastolic blood pressure values from the average of the monitor diastolic blood pressure values.

According to another preferred feature of the present invention, the BP monitor further comprising a blood pressure measuring device which increases the pressure of the cuff up to a target pressure which is higher than a systolic blood pressure of the subject and measures at least one of a systolic, a mean, and a diastolic blood pressure value of the living subject based on a variation of respective amplitudes of pulses of a pulse wave which are produced in the cuff and detected by the pressure sensor during at least one of the increasing of the cuff pressure up to the target pressure and a decreasing of the cuff pressure down from the target pressure. The target pressure may be, e.g., about 180 mmHg that is estimated to be sufficiently higher than a normal systolic BP value of a human being.

According to another preferred feature of the present invention, the blood pressure measuring device comprises means for measuring the at least one of the systolic, the mean, and the diastolic blood pressure value of the living subject when the abnormality identifying means identifies the abnormality.

According to another preferred feature of the present invention, the BP monitor further comprising a display which displays the monitor diastolic blood pressure value determined by the blood-pressure determining means.

### BRIEF DESCRIPTION OF THE DRAWINGS

The above and optional objects, features, and advantages of the present invention will better be understood by reading the following detailed description of the preferred embodiments of the invention when considered in conjunction with the accompanying drawings, in which:
Fig. 1 is a diagrammatic view of a blood pressure (BP) monitor embodying the present invention;
Fig. 2 is an illustrative view for explaining various functions of the BP monitor of Fig. 1;
Fig. 3 is a flow chart representing a main routine which is executed by a control device of the BP monitor of Fig. 1;
Fig. 4 is a flow chart representing a BP monitor routine as a step of the flow chart of Fig. 3;
Fig. 5A is a time chart showing a relationship between time and cuff pressure, P_{c}, or pulse amplitude, A;
Fig. 5B is a table showing a manner in which a cuff pressure, P_{c}, is determined as a monitor diastolic blood pressure, MBP_{DIA}; and
Fig. 6 is a graph showing the envelope of pulse amplitudes obtained by changing cuff pressure applied to a living subject having a normal blood pressure, and the envelope of pulse amplitudes obtained by changing cuff pressure applied to a living subject having a low blood pressure.

### DETAILED DESCRIPTION OF THE PREFERRED EMBODIMENTS

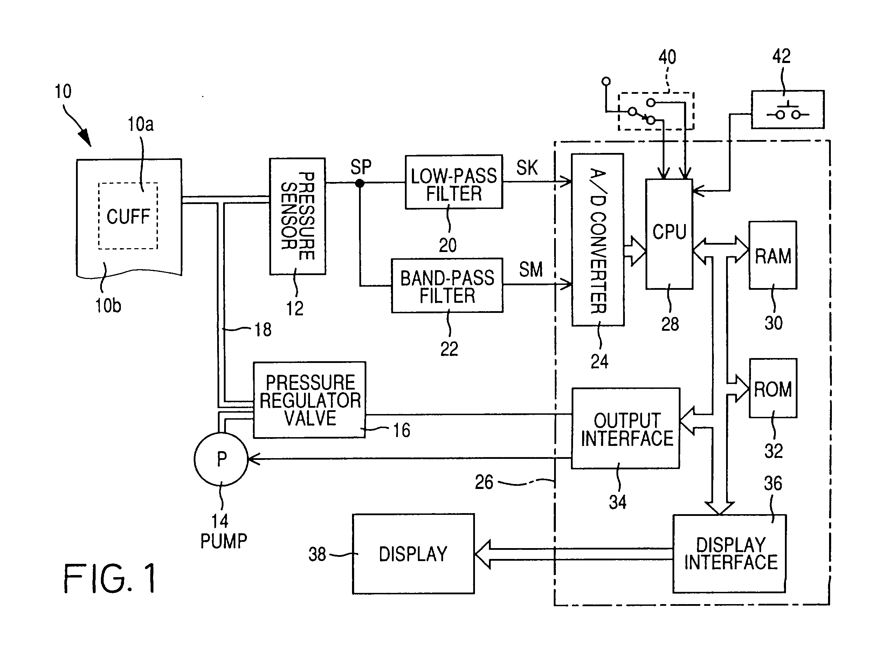

Referring to Figs. 1 through 5 (5A and 5B), there will be described a blood pressure (BP) monitor to which the present invention is applied.

In Fig. 1, reference numeral 10 designates an inflatable cuff which is adapted to be wound around an upper arm of a living subject, such as a patient. The cuff 10 is provided by an inflatable bag 10a formed of a resilient sheet such as a rubber sheet or a vinyl sheet, and a non-stretchable arm belt 10b in which the bag 10a is accommodated. The bag 10a is connected via air piping 18 to a pressure sensor 12, an air pump 14, and a pressure regulator valve 16.

The pressure sensor 12 includes a pressure-sensing semiconductor element which detects an air pressure in the cuff 10 (i.e., bag 10a), generates a pressure signal, SP, representing the detected cuff pressure, and supplies the pressure signal SP to each of a low-pass filter 20 and a band-pass filter 22. The low-pass filter 20 extracts, from the pressure signal SP, a direct-current (DC) component representing a static pressure, P_{c}, of the cuff 10, and supplies a cuff-pressure signal, SK, representing the static pressure P_{c}, to an analog-to-digital (A/D) converter 24. The band-pass filter 22 extracts, from the pressure signal SP, an alternate-current (AC) or frequency (e.g., 1 to 10 Hz) component representing pulses of a pulse wave which are produced in the cuff 10. The band-pass filter 22 supplies a pulse-wave signal, SM, representing the pulse wave, to the A/D converter 24. The pulse wave is a pressure oscillation which is transmitted from the arteries of the living subject to the cuff 10 in synchronism with the heartbeat of the subject and is produced in the cuff 10.

The band-pass filter 22 functions as a pulse-wave sensor, and has a frequency characteristic that the band width thereof is sufficiently narrow to be able to extract, freely from noise such as motion artifact noise, the respective amplitudes of pulses of a pulse wave, i.e., pressure oscillation that is produced in the cuff 10 in synchronism with the heartbeat of the living subject, while the pressure of the cuff 10 is slowly decreased at the rate of 2 to 3 mmHg/sec. The A/D converter 24 includes a multiplexer which processes the two input signals SK, SM by time sharing, and has the function of concurrently converting the two analog signals into digital signals SK, SM, which are supplied to a central processing unit (CPU) 28 of a control device 26.

The control device 26 is provided by a microcomputer including the CPU 28, a random access memory (RAM) 30, a read only memory (ROM) 32, an output interface 34, and a display interface 36. The CPU 28 processes the input signals SK, SM supplied from the A/D converter 24, by utilizing a temporary-storage function of the RAM 30, according to control programs pre-stored in the ROM 32. The CPU 28 controls the air pump 14 and the pressure regulator valve 16 via the output interface 34, and controls a display 38 via the display interface 36. The display 38 includes an image display panel (not shown) which displays an image, such as BP values and waveforms, consisting of a number of picture elements, and a printer (not shown) which records, on a recording sheet of paper, using an ink, the image currently displayed on the image display panel. In the present embodiment, the air pump 14, the pressure regulator valve 16, and the control device 26 cooperate with one another to provide a cuff-pressure regulating device 52 which will be described later.

A mode switch 40 is manually operable by a user for selectively establishing a BP measure mode and a BP monitor mode. The mode switch 40 supplies a mode signal indicative of the selected mode, to the CPU 28. A Start/Stop switch 42 is manually operable by the user for inputting a start command or a stop command to start or stop an operation of the present BP monitor, and supplies a command signal indicative of the input command, to the CPU 28.

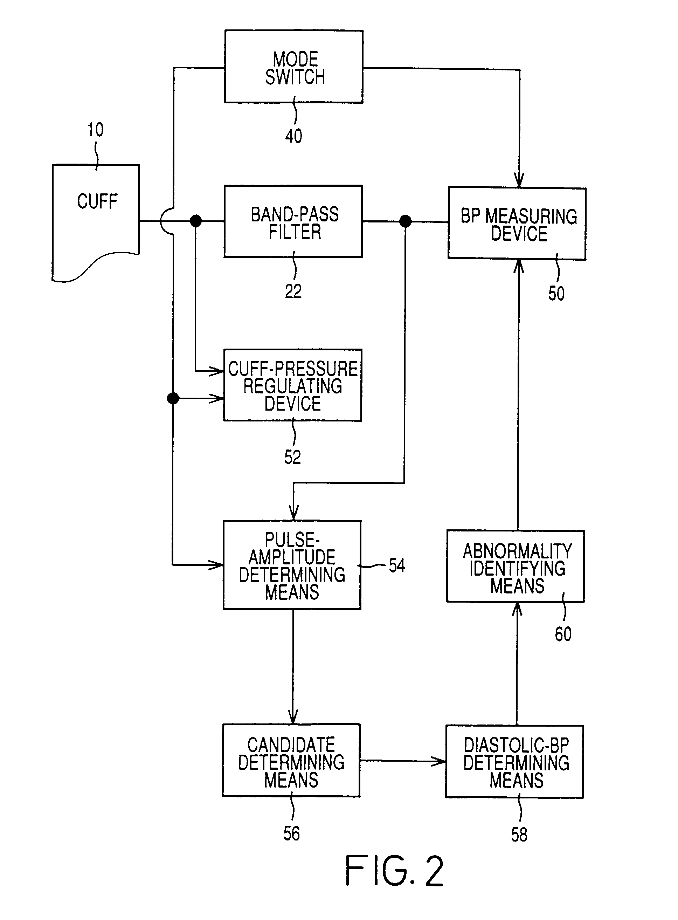

Fig. 2 illustratively shows the various functions of the present BP monitor. When the mode switch 40 is operated to select the BP measure mode, the cuff-pressure regulating device 52 is operated to increase the pressure of the cuff 10 (hereinafter, referred to as the "cuff pressure P_{c}") up to a target pressure higher than a systolic BP value of the subject. While the cuff pressure is increased, or subsequently decreased from the target pressure at a low rate of 2 to 3 mmHg/sec, a BP measuring device 50 carries out an oscillometric BP measuring method in which a BP value of the subject is measured based on the variation of respective amplitudes, Aₙ (n are natural numbers), of a series of pulses of a pulse wave which is detected from the cuff 10. The BP measuring device 50 is provided by the pressure sensor 12, the low-pass filter 20, the band-pass filter 22, and the control device 26. The BP measuring device 50 performs a BP measurement in the case where the operation of the BP monitor is started in the BP measure mode. The BP measuring device 50 also performs a BP measurement when abnormality identifying means 60 (described later) identifies an abnormal change of monitor diastolic BP values determined by diastolic-BP determining means 58 (described later). The control device 26 functions as both the diastolic-BP determining means 58 and the abnormality identifying means 60.

In the BP monitor mode, the cuff-pressure regulating device 52 operates, at a predetermined interval of time, for increasing the cuff pressure at a predetermined rate of change. Pulse-amplitude determining means 54 determines, by calculation, respective amplitudes, Aₘ (m are natural numbers), of pulses of a pulse wave which is produced in the cuff 10 in synchronism with the heartbeat of the subject while the cuff pressure is increased by the cuff-pressure regulating device 52. Candidate determining means 56 judges whether an amplitude Aₘ (m ≤ i - 1) of each of prior pulses determined by the pulse-amplitude determining means 54 is not greater than a reference value, Aₐ (= Aₘ x (1 - R), where m = i and 0 < R < 1) which is smaller than an amplitude Aₘ (m = i) of a subsequent pulse determined by the pulse-amplitude determining means 54, by a predetermined proportion, R, of the amplitude Aₘ of the subsequent pulse. The amplitude of each of the prior pulses is determined before the amplitude of the subsequent pulse is determined. If a positive judgment is made, the candidate determining means 56 determines, as a diastolic BP candidate, P_{ck}, of the subject, a cuff pressure P_{c} detected when each prior pulse is detected in the cuff 10. The control device 26 functions as both the pulse- amplitude determining means 54 and the candidate determining means 56. The diastolic-BP determining means 58 determines, as a monitor diastolic BP value, MBP_{DIA}, of the subject, the cuff pressure P_{c} corresponding to the amplitude of a prior pulse, if the candidate determining means 56 determines, as a diastolic BP candidate, the cuff pressure corresponding to the amplitude of that prior pulse, with respect to each of a predetermined number, N₀, of the subsequent pulses.

The abnormality identifying means 60 identifies an abnormality of the monitor diastolic BP values MBP_{DIA} determined by the diastolic-BP determining means 58, such as an abrupt decrease of the blood pressure of the subject. If the identifying means 60 identifies an abnormality, the BP measuring device 50 is immediately operated for carrying out an oscillometric BP measurement.

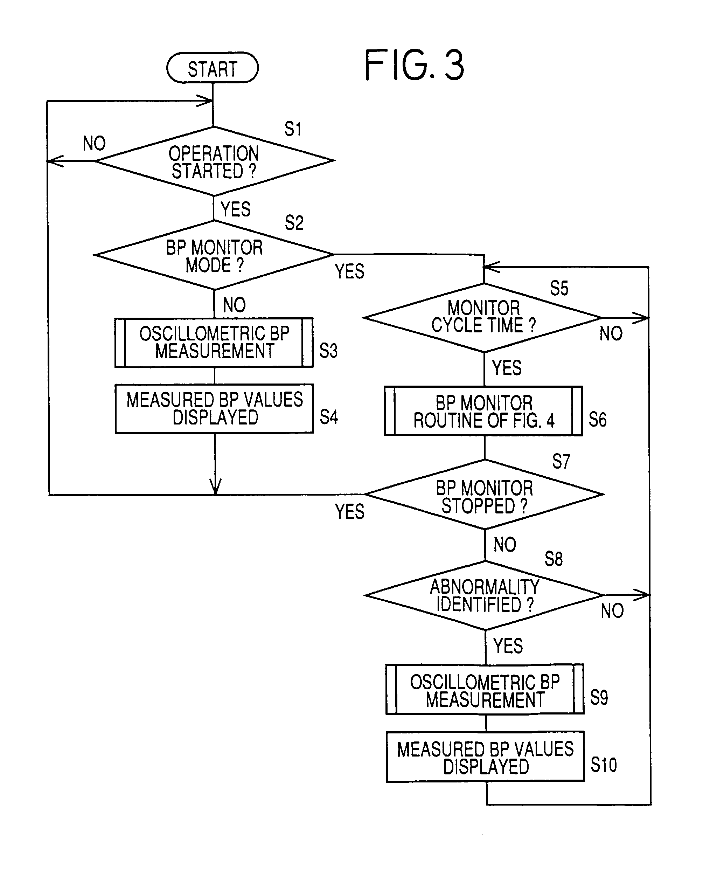

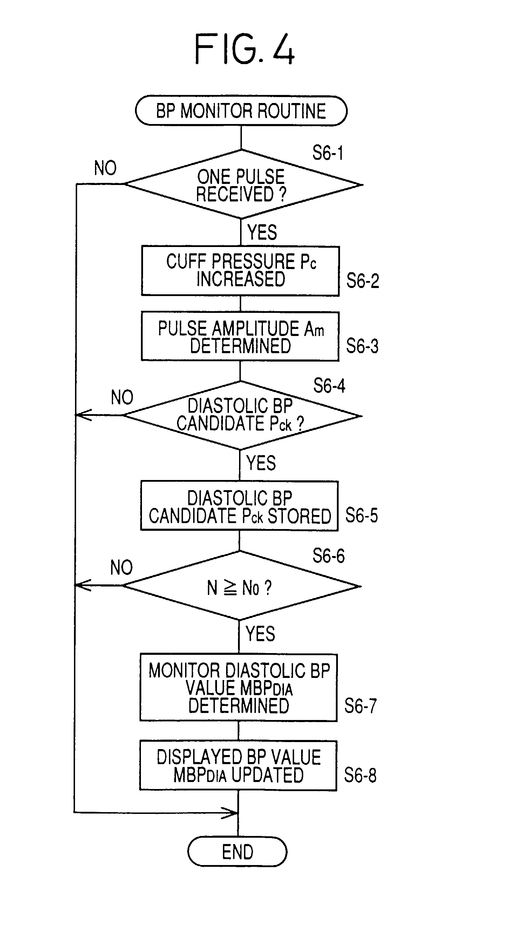

Next, there will be described the operation of the present BP monitor by reference to the flow charts of Figs. 3 and 4.

First, at Step S1, the CPU 28 judges, based on the command signal supplied from the Start/Stop switch 42, whether the Start/Stop switch 43 has been operated to input a start command to start the operation of the BP monitor. If a negative judgment is made at Step S1, the control of the CPU 28 repeats Step S1 while waiting for a positive judgment to be made at Step S1. Meanwhile, if a positive judgment is made, the control of the CPU 28 proceeds with Step S2 to judge whether the mode switch 40 has been operated to select the BP monitor mode. In the case where the BP measure mode is in use, a negative judgment is made at Step S2, so that the control of the CPU 28 goes to Step S3, i.e., BP measure routine according to which a known oscillometric BP measurement is carried out to measure a systolic, a diastolic, and a mean BP value, BP_{SYS}, BP_{DIA}, BP_{MEAN}, of the subject. When the BP measurement is finished, the pressure regulator valve 16 is opened to quickly deflate the cuff 10, thereby releasing the upper arm of the subject from the cuff pressure, i.e., pressing force of the cuff 10. Step S3 is followed by Step S4 to store the measured BP values in the RAM 30 and operate the display 38 to indicate numerals representing the measured BP values.

More specifically described, in the oscillometric BP measurement effected at Step S3, the air pump 14 and the pressure regulator valve 16 are operated to quickly increase the cuff pressure P_{c} up to a predetermined target pressure, P_{cm}, e.g., 180 mmHg. Subsequently, the air pump 14 is stopped and the degree of opening of the regulator valve 16 is regulated, so that a slow deflation of the cuff 10 is started. That is, the cuff pressure P_{c} is decreased at a low rate of 2 to 3 mmHg/sec which is suitable for BP measurements. During this slow cuff-pressure decrease, the control device 26 determines BP values according to a well known oscillometric BP determining algorithm. That is, the CPU 28 determines, as a systolic BP value BP_{SYS}, a cuff pressure at the time when the pulse amplitudes Aₙ significantly change in a phase in which the amplitudes Aₙ increase; determines, as a mean BP value BP_{MEAN}, a cuff pressure at the time when the amplitudes Aₙ take a maximum value, i.e., when a pulse having a maximum amplitude is produced; and determines, as a diastolic BP value BP_{DIA}, a cuff pressure at the time when the pulse amplitudes Aₙ significantly change in a phase in which the amplitudes Aₙ decrease.

In the case where the mode switch 40 has been operated to select the BP monitor mode, a positive judgment is made at Step S2 and accordingly the control of the CPU 28 goes to Step S5 to judge whether a timer has counted up a predetermined monitor cycle time from the time when a monitor diastolic BP value MBP_{DIA} had been determined at Step S6 in the preceding control cycle in accordance with the main routine of Fig. 3. The monitor cycle time may fall in the range of from several minutes to ten and several minutes. If a negative judgment is made at Step S5, the CPU 28 repeats Step S5 until a positive judgment is made. Meanwhile, if a positive judgment is made at Step S5, the control of the CPU 28 goes to Step S6, i.e., BP monitor routine of Fig. 4.

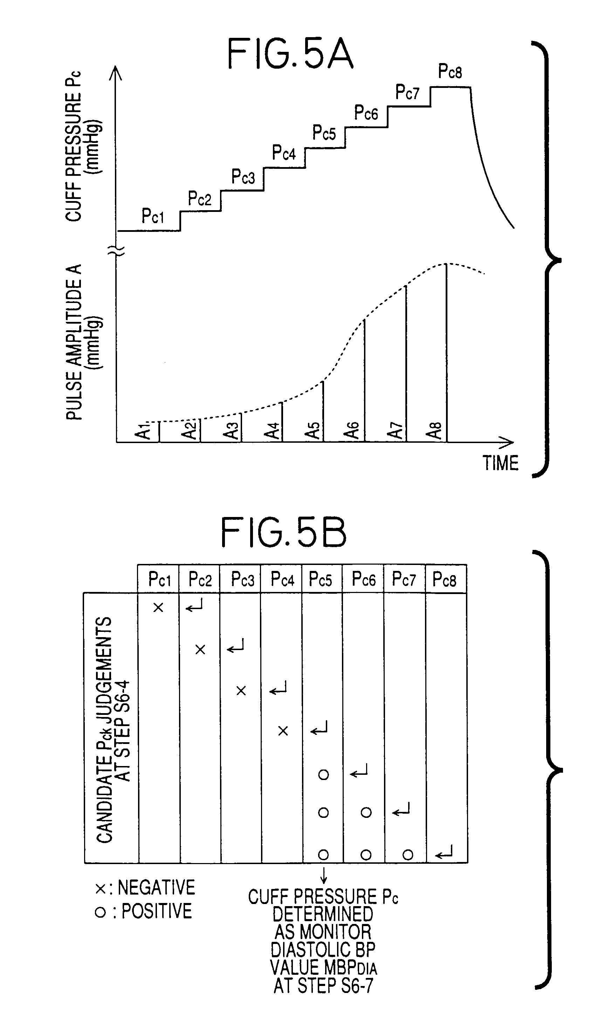

At Step S6-1 of Fig. 4, the CPU 28 judges whether the CPU 28 has received, from the band-pass filter 22, a pulse-wave signal SM representing one pulse of a pulse wave. If a negative judgment is made at Step S6-1, the current control cycle in accordance with the routine of Fig. 4 is ended. On the other hand, if a positive judgment is made at Step S6-1, the control of the CPU 28 goes to Step S6-2 to increase the cuff pressure P_{c} by a predetermined pressure increase amount, ΔP_{c}, and subsequently to Step S6-3 to determine, by calculation, an amplitude Aₘ of the pulse received at Step S6-1 and store, in an appropriate area of the RAM 30, the determined amplitude Aₘ together with the cuff pressure P_{c} at the time when the pulse is detected through the band-pass filter 22. The cuff pressure P_{c} is read from the cuff-pressure signal SK supplied from the low-pass filter 20. While Steps S6-2 and S6-3 are repeated, the cuff pressure P_{c} is increased at a predetermined rate of change, more specifically, stepwise by the respective pressure amounts ΔP_{c}, as shown in Fig. 5. While the cuff pressure P_{c} is held for a short duration at each of the pressure steps, the CPU 28 reads in one pulse, determines the amplitude Aₘ of the pulse, and stores the pulse amplitude Aₘ in the RAM 30. Step S6-3 corresponds to the pulse-amplitude determining means 54.

At Step S6-4, the CPU 28 judges whether the amplitude Aₘ (m ≤ i - 1) of each of the prior pulses determined at Step S6-3 in the prior control cycles before the current control cycle is not greater than a reference value Aₐ (e.g., 0.7 x Aₘ) which is smaller than the amplitude Aₘ (m = i) of the current pulse determined at Step S6-3 in the current control cycle, by a predetermined proportion R (e.g., 30 % (R= 0.3)) of the amplitude Aₘ of the current pulse. If a positive judgment is made, the CPU 28 determines, as a diastolic BP candidate P_{ck} of the subject, the cuff pressure P_{c} corresponding to the pulse amplitude Aₘ of each prior pulse. While the cuff pressure P_{c} is increased up to a mean BP value BP_{MEAN} of the subject, the pulse amplitudes Aₘ continue to increase as indicated at broken line in Fig. 5A. For example, in the case where the pulse amplitude A₂ is determined at Step S6-3, the only prior amplitude A₁ is compared with 0.7 x (the amplitude A₂). If the amplitude A₁ is greater than 0.7 x (the amplitude A₂), the cuff pressure P_{c1} corresponding to the amplitude A₁ is not determined as a diastolic BP candidate P_{ck}. In the example shown in Fig. 5B, a negative judgment is made at Step S6-4 for each of the amplitudes A₁ to A₄. Once a negative judgment is made for a pulse amplitude Aₘ at Step S6-4, the CPU 28 never makes a judgment for that amplitude at Step S6-4 in the following control cycles.

On the other hand, if the CPU 28 makes a positive judgment at Step S6-4, the control of the CPU 28 goes to Step S6-5 to determine the cuff pressure P_{c} corresponding to the pulse amplitude Aₘ, as a diastolic BP candidate P_{ck}, and store the pressure P_{c} in an appropriate area of the RAM 30. For example, in the case where the pulse amplitude A₆ is determined at Step S6-3, the prior amplitude A₅ is compared with 0.7 x (the amplitude A₆) and, if the amplitude A₅ is not greater than 0.7 x (the amplitude A₆), the cuff pressure P_{c5} corresponding to the amplitude A₅ is determined as a diastolic BP candidate P_{ck}. Thus, a positive judgment is made at Step S6-4 for the amplitude A₅. Regarding the example shown in Fig. 5B, a positive judgment is made for the amplitude A₅, when the amplitude A₅ is compared with the amplitude A₇ in the next cycle, and with the amplitude A₈ in the cycle after that next cycle. Steps S6-4 and S6-5 correspond to the candidate determining means 56.

Step S6-5 is followed by Step S6-6 to judge, regarding each of the prior amplitudes Aₘ, whether a number, N, of the positive judgments made for each prior amplitude Aₘ at Step S6-4 becomes not smaller than a reference number, N₀, e.g., 3. If a positive judgment is made for any of the prior amplitudes Aₘ, a positive judgment is finally made at Step S6-5. For example, in the example shown in Fig. 5B, when the amplitude A₈ is determined at Step S6-3, a positive judgment is made for the amplitude A₅ and a negative judgment is made for each of the amplitudes A₆ and A₇. Accordingly, a positive judgment is finally made at Step S6-6. The reference number N₀ is empirically determined as being suitable for obtaining a diastolic BP value. In the case where the above-indicated pressure increase amount ΔPc is about 15 mmHg, the reference number N₀ is determined at 3.

If a negative judgment is finally made at Step S6-6, i.e., if a negative judgment is made for all the prior amplitudes Aₘ, the control of the CPU 28 goes back to Step S6-1. On the other hand, if a positive judgment is finally made at Step S6-6, the control goes to Step S6-7 to determine, as a monitor diastolic BP value MBP_{DIA}, the cuff pressure P_{c} corresponding to the pulse amplitude Aₘ for which three positive judgments are made at Step S6-4. The thus determined cuff pressure P_{c} is stored in the RAM 30. Regarding the example shown in Fig. 5B, the cuff pressure P_{c5} is determined as a monitor diastolic BP value MBP_{DIA}. In the present embodiment, Steps S6-6 and S6-7 correspond to the diastolic-BP determining means 58. Step S6-7 is followed by Step S6-8 to display the newly determined monitor diastolic BP value MBP_{DIA} in place of the old value MBP_{DIA} determined in Step S6 in the preceding control cycle in accordance with the main routine of Fig. 3.

After the monitor diastolic BP value MBP_{DIA} is determined at Step S6, the control of the CPU 28 goes to Step S7 to judge whether the Start/Stop switch 42 is operated to stop the BP monitoring operation. If a positive judgment is made at Step S7, the control goes back to Step S1. On the other hand, if a negative judgment is made at Step S8, the control goes to Step S8 to judge whether an abnormality has occurred to the monitor diastolic BP values MBP_{DIA}. For example, the CPU 28 identifies an abnormality of the diastolic BP values MBP_{DIA}, if an amount, or a rate, of change of the current value MBP_{DIA} from a moving average of all the prior values MBP_{DIA} exceeds a reference value, which indicates that the blood pressure of a living subject has abruptly decreased. Step S8 corresponds to the abnormality identifying means 60.

If a negative judgment is made at Step S8, the control goes back to Step S5 and repeats Steps S5 to S8. Meanwhile, if a positive judgment is made at Step S8, the control of the CPU 28 goes to Step S9 to carry out an oscillometric BP measurement using the cuff 10 like at Step S3. Step S9 is followed by Step S10 to operate the display 38 to display the measured BP values BP_{SYS}, BP_{MEAN}, BP_{DIA}.

As is apparent from the foregoing description, the present BP monitor operates such that in the BP monitor mode the amplitudes Aₘ of pulses of a pulse wave which are produced in the cuff 10 while the cuff pressure P_{c} is increased by the cuff-pressure regulating device 52, are determined by the pulse-amplitude determining means 54. In addition, the candidate determining means 56 judges whether the amplitude Aₘ (m ≤ i - 1) of each of the prior pulses determined in the prior control cycles before the current control cycle is not greater than the reference value Aₐ which is smaller than the amplitude Aₘ (m = i) of the current pulse determined in the current control cycle, by the predetermined proportion R of the amplitude Aₘ of the current pulse and, if a positive judgment is made, determines, as a diastolic BP candidate P_{ck} of the subject, the cuff pressure P_{c} corresponding to the amplitude Aₘ of each prior pulse. The diastolic-BP determining means 58 judges, regarding each of the prior amplitudes Aₘ, whether the number N of the positive judgments made for each prior amplitude Aₘ becomes not smaller than the reference number N₀ and, if a positive judgment is made for any of the prior amplitudes Aₘ, determines, as a monitor diastolic BP value MBP_{DIA}, the cuff pressure P_{c} corresponding to the prior amplitude Aₘ for which the predetermined number N₀ of positive judgments are made. The present BP monitor has been developed based on the fact that a diastolic BP value BP_{DIA} does not correspond to a pulse having an amplitude not smaller than an amplitude, Aₘₐₓ x (1 - R), smaller than a maximum amplitude, Aₘₐₓ, of the last pulse that is detected in the current control cycle, by the predetermined proportion R of the amplitude Aₘₐₓ of the last pulse. The last or current pulse has a maximum amplitude Aₘₐₓ of all the amplitudes Aₘ of the prior pulses which have been detected prior to the last or current pulse while the cuff pressure P_{c} is increased, as indicated in Fig. 5A. In addition, the present BP monitor has been developed based on the fact that an amplitude of a pulse correctly corresponding to a diastolic BP value BP_{DIA} does not change even if the cuff pressure P_{c} is increased.

Therefore, the present BP monitor can determine a monitor diastolic BP value at a pressure level higher than the diastolic BP value by only the product of the pressure increase amount ΔP_{c} and the reference number N₀. This monitor diastolic BP value enjoys high accuracy. In addition, since the pressure level where the BP value is determined is considerably low, the living subject does not feel discomfort.

In the present embodiment, the cuff-pressure regulating device 52 stepwise increases the cuff pressure P_{c}, by alternately increasing it by the increment amount ΔP_{c} and holding it at each increased level. The pulse-amplitude determining means 54 determines, by calculation, the amplitude Aₘ of the pulse which is produced when the cuff pressure P_{c} is held at each increased level. The thus determined pulse amplitude Aₘ enjoys high accuracy because it is free from the adverse influence resulting from the increasing of the cuff pressure P_{c}. Therefore, the monitor BP values are determined with accuracy based on the pulse amplitudes Aₘ.

In addition, in the present embodiment, when the abnormality identifying means 60 identifies an abnormality of the monitor diastolic BP values MBP_{DIA}, the BP monitor automatically carries out an oscillometric BP measurement by increasing the cuff pressure P_{c} up to a high level which is estimated to be higher than a systolic BP value of a living subject. Thus, the BP monitor provides accurate BP values upon identification of an abnormality of the subject. Therefore, a doctor or a nurse can take an appropriate medical treatment on the subject.

While the present invention has been described in its preferred embodiment, the present invention may otherwise be embodied.

For example, although in the illustrated embodiment the pressure P_{c} of the cuff 10 is stepwise increased at a predetermined rate in the BP monitor mode, it is possible that the cuff pressure P_{c} be continuously increased at a predetermined rate.

While in the illustrated embodiment the pressure increase amount ΔP_{c} is a constant value, it is possible that the pressure increase amount ΔP_{c} be variable depending upon the current cuff pressure P_{c}.

Although in the illustrated embodiment the predetermined proportion R used at Step S6-4 is a constant value, it is possible that the CPU 28 determine a value R based on the variation of the amplitudes of pulses of a pulse wave obtained in the oscillometric BP measurement effected at Step S3. In the latter case, the BP monitor can determine a value R suitable for each individual subject.

While in the illustrated embodiment the reference number N₀ used at Step S6-6 is a constant value, it is possible that the CPU 28 determine a number N₀ based on the variation of the amplitudes of pulses of a pulse wave obtained in the oscillometric BP measurement effected at Step S3. In the latter case, the BP monitor can determine a number N₀ suitable for each individual subject.

In the illustrated embodiment, the BP measuring device 50 performs an oscillometric BP measurement at Step S3 when the operation of the BP monitor is started in a state in which the BP measure mode has been selected, or the abnormality identifying means 60 identifies an abnormality of the monitor diastolic BP values determined by the diastolic-BP determining means 58. However, it is possible to adapt the BP measuring device 50 to periodically perform an oscillometric BP measurement at a predetermined cycle time, i.e., at a predetermined interval of time.

It is to be understood that the present invention may be embodied with other changes, improvements, and modifications that may occur to those skilled in the art without departing from the scope of the invention defined in the appended claims.

## Claims

1. A blood pressure monitor comprising:
an inflatable cuff (10) which is adapted to be wound around a body portion of a living subject to press said body portion;
a pressure sensor (12) which detects a pressure in said cuff;
a cuff-pressure regulating device (52) which increases the pressure of said cuff;
pulse-amplitude determining means (54) for determining an amplitude (Aₘ) of each of pulses of a pulse wave (SM) which are produced in said cuff and detected by said pressure sensor while the pressure of said cuff is increased by said cuff-pressure regulating device; characterised by
candidate determining means (56) for determining, as a diastolic blood pressure candidate (P_{ck}), a pressure (P_{c}) of said cuff which is detected by said pressure sensor and which corresponds to an amplitude of a first pulse of said pulses determined by said pulse-amplitude determining means, by judging whether the amplitude of said first pulse is not greater than a reference value which is smaller than an amplitude of at least one second pulse of said pulses, by a predetermined proportion of the amplitude of said second pulse, the amplitude of said second pulse being determined by said pulse-amplitude determining means after the amplitude of said first pulse is determined; and
blood-pressure determining means (58) for determining, as a monitor diastolic blood pressure value (MBP_{DIA}), said pressure of said cuff corresponding to said amplitude of said first pulse, when said candidate determining means determines, as said diastolic blood pressure candidate, said pressure of said cuff corresponding to said amplitude of said first pulse, with respect to a predetermined number of said at least one second pulse.

2. A blood pressure monitor according to claim 1, wherein said candidate determining means (56) comprises judging means (S6-6) for judging whether the amplitude of said first pulse is not greater than a reference value which is smaller than an amplitude of each of a plurality of second pulses of said pulses, by a predetermined proportion of the amplitude of said each second pulse, the respective amplitudes of said second pulses being determined by said pulse-amplitude determining means after the amplitude of said first pulse is determined.

3. A blood pressure monitor according to claim 1 or claim 2, wherein said cuff-pressure regulating device (52) comprises pressure increasing means (S6-2) for stepwise increasing the pressure of said cuff by alternately increasing the cuff pressure and maintaining the cuff pressure at each of a plurality of different pressure values, and wherein said pulse-amplitude determining means (54) determines an amplitude of at least one pulse which is produced in said cuff and detected by said pressure sensor while the cuff pressure is maintained at said each pressure value.

4. A blood pressure monitor according to any of claims 1 to 3, wherein said blood-pressure determining means comprises monitor means (S5) for iteratively determining said monitor diastolic blood pressure value.

5. A blood pressure monitor according to claim 4, further comprising abnormality identifying means (60) for identifying an abnormality of the monitor diastolic blood pressure values iteratively determined by said monitor means.

6. A blood pressure monitor according to claim 5, wherein said abnormality identifying means comprises means (S8) for identifying said abnormality based on at least one of an amount of change of a last determined value of said monitor diastolic blood pressure values from an average of said monitor diastolic blood pressure values, and a rate of change of said last determined value of said monitor diastolic blood pressure values from said average of said monitor diastolic blood pressure values.

7. A blood pressure monitor according to claim 5 or claim 6, further comprising a blood pressure measuring device (10, 12, S3) which increases the pressure of said cuff up to a target pressure which is higher than a systolic blood pressure of the subject and measures at least one of a systolic, a mean, and a diastolic blood pressure value of the living subject based on a variation of respective amplitudes of pulses of a pulse wave which are produced in said cuff and detected by said pressure sensor during at least one of the increasing of the cuff pressure up to said target pressure and a decreasing of the cuff pressure down from said target pressure.

8. A blood pressure monitor according to claim 7, wherein said blood pressure measuring device comprises means (S9) for measuring said at least one of said systolic, said mean, and said diastolic blood pressure value of the living subject when said abnormality identifying means identifies said abnormality.

9. A blood pressure monitor according to any of claims 1 to 8, further comprising a display (38) which displays said monitor diastolic blood pressure value determined by said blood-pressure determining means.

10. A blood pressure monitor according to any of claims 1 to 9, wherein said predetermined proportion is 0.3.

11. A blood pressure monitor according to any of claims 1 to 10, wherein said predetermined number is three.

## Patentansprüche

1. Blutdruckmonitor mit:
einer aufblasbaren Manschette (10), welche angepasst ist, um um einen Körperabschnitt eines lebenden Wesens gewickelt zu werden, um den Körperabschnitt zu pressen;
einem Drucksensor (12), welcher einen Druck in der Manschette erfasst;
einer Manschettendruckreglervorrichtung (52), welche den Druck der Manschette erhöht;
einer Pulsamplitudenbestimmungseinrichtung (54), welche eine Amplitude (Aₘ) jedes Pulses einer Pulswelle (SM) erfasst, welche in der Manschette erzeugt und von dem Drucksensor erfasst werden, während der Druck der Manschette durch die Manschettendruckreglervorrichtung erhöht wird; gekennzeichnet durch
eine Kandidatenbestimmungseinrichtung (56), welche als diastolischen Blutdruckkandidaten (P_{ck}) einen Druck (P_{c}) der Manschette, welcher durch den Drucksensor erfasst wird und einer Amplitude eines ersten Pulses der Pulse entspricht, welche von der Pulsamplitudenbestimmungseinrichtung bestimmt werden, durch Beurteilen bestimmt, ob die Amplitude des ersten Pulses nicht größer als ein Bezugswert, der kleiner als eine Amplitude wenigstens eines zweiten Pulses der Pulse ist, um einen vorbestimmten Anteil der Amplitude des zweiten Pulses ist, wobei die Amplitude des zweiten Pulses durch die Pulsamplitudenbestimmungseinrichtung bestimmt wird, nachdem die Amplitude des ersten Pulses bestimmt worden ist; und
eine Blutdruckbestimmungseinrichtung (58), welche als zu überwachenden diastolischen Blutdruckwert (MBP_{DIA}) den Druck der Manschette entsprechend der Amplitude des ersten Pulses bestimmt, wenn die Kandidatenbestimmungseinrichtung als den diastolischen Blutdruckkandidaten den Druck der Manschette entsprechend der Amplitude des ersten Pulses bezüglich einer vorbestimmten Zahl von wenigstens einem zweiten Puls bestimmt.

2. Blutdruckmonitor nach Anspruch 1, dadurch gekennzeichnet, dass die Kandidatenbestimmungseinrichtung (56) eine Beurteilungseinrichtung (S6-6) zum Beurteilen aufweist, ob die Amplitude des ersten Pulses nicht größer als ein Bezugswert, welcher kleiner als eine Amplitude von jedem einer Mehrzahl von zweiten Pulsen der Pulse ist, um einen vorbestimmten Anteil der Amplitude jedes zweiten Pulses ist, wobei die jeweiligen Amplituden der zweiten Pulse durch die Pulsamplitudenbestimmungseinrichtung bestimmt wird, nachdem die Amplitude des ersten Pulses bestimmt worden ist.

3. Blutdruckmonitor nach Anspruch 1 oder 2, dadurch gekennzeichnet, dass die Manschettendruckreglervorrichtung (52) eine Druckerhöhungseinrichtung (S6-6) aufweist, welche stufenweise den Druck der Manschette durch abwechselndes Erhöhen des Manschettendrucks erhöht und den Manschettendruck an jedem einer Mehrzahl von unterschiedlichen Druckwerten aufrechterhält, und die Pulsamplitudenbestimmungseinrichtung (54) einen Amplitude wenigstens eines Pulses bestimmt, welcher in der Manschette erzeugt und durch den Drucksensor erfasst wird, während der Manschettendruck unter jedem Druckwert aufrechterhalten wird.

4. Blutdruckmonitor nach einem der Ansprüche 1 bis 3, dadurch gekennzeichnet, dass die Blutdruckbestimmungseinrichtung eine Monitoreinrichtung (S5) aufweist, welche den zu überwachenden diastolischen Blutdruckwert iterativ bestimmt.

5. Blutdruckmonitor nach Anspruch 4, des weiteren gekennzeichnet durch eine Abnormalitätsidentifizierungseinrichtung (60), welche eine Abnormalität der zu überwachenden diastolischen Blutdruckwerte identifiziert, welche von der Überwachungseinrichtung bestimmt werden.

6. Blutdruckmonitor nach Anspruch 5, dadurch gekennzeichnet, dass die Abnormalitätsidentifizierungseinrichtung eine Einrichtung aufweist, welche die Abnormalität auf der Grundlage wenigstens eines Änderungsbetrags eines zuletzt bestimmten Werts der zu überwachenden diastolischen Blutdruckwerte aus einem Durchschnitt der zu überwachenden diastolischen Blutdruckwerte und eine Änderungsrate des zuletzt bestimmten Werts der zu überwachenden diastolichen Blutdruckwerte aus dem Durchschnitt der zu überwachenden diastolischen Blutdruckwerte identifiziert.

7. Blutdruckmonitor nach Anspruch 5 oder 6, des weiteren gekennzeichnet durch eine Blutdruckmessvorrichtung (10, 12, S3), welche den Druck der Manschette bis hin zu einem Zieldruck erhöht, welcher höher als ein systolischer Blutdruck des Wesens ist, und wenigstens einen systolischen, einen mittleren oder einen diastolischen Blutdruckwert des lebenden Wesens auf der Grundlage einer Änderung von jeweiligen Amplituden von Pulsen einer Pulswelle misst, welche wenigstens während des Erhöhens des Manschettendrucks bis auf den Zieldruck oder eines Verringerns des Manschettendrucks ab dem Zieldruck in der Manschette erzeugt und durch den Drucksensor erfasst werden.

8. Blutdruckmonitor nach Anspruch 7, dadurch gekennzeichnet, dass die Blutdruckmessvorrichtung eine Einrichtung (S9) aufweist, welche wenigstens den systolischen, den mittleren oder den diastolischen Blutdruckwert des lebenden Wesens misst, wenn die Abnormalitätsidentifizierungseinrichtung die Abnormalität identifiziert.

9. Blutdruckmonitor nach einem der Ansprüche 1 bis 8, des weiteren gekennzeichnet durch eine Anzeige (38), welche den zu überwachenden diastolischen Blutdruckwert anzeigt, der von der Blutdruckbestimmungseinrichtung bestimmt wird.

10. Blutdruckmonitor nach einem der Ansprüche 1 bis 9, dadurch gekennzeichnet, dass der vorbestimmte Anteil 0,3 ist.

11. Blutdruckmonitor nach einem der Ansprüche 1 bis 10, dadurch gekennzeichnet, dass die vorbestimmte Zahl 3 ist.

## Revendications

1. Dispositif de surveillance de la pression sanguine, comprenant :
un brassard (10) gonflable, propre à être enroulé autour d'une partie corporelle d'un être vivant afin de comprimer cette partie du corps ;
un capteur de pression (12), qui détecte une pression dans ledit brassard ;
un dispositif de réglage de pression de brassard (52), qui augmente la pression exercée par ledit brassard ;
un moyen de détermination d'amplitudes d'impulsions (54) servant à déterminer une amplitude (Aₘ) de chacune des impulsions d'une onde impulsionnelle (SM), qui sont produites dans ledit brassard et détectées par ledit capteur de pression tandis que la pression exercée par ledit brassard est augmentée par ledit moyen de réglage de pression de brassard ;
caractérisé par
un moyen de détermination de valeurs candidates (56) afin de déterminer, en tant que valeur candidate de la pression sanguine diastolique (P_{ck}), une pression (P_{c}) dudit brassard, qui est détectée par ledit capteur de pression et qui correspond à une amplitude d'une première impulsion desdites impulsions déterminées par ledit moyen de détermination d'amplitudes d'impulsions, en établissant si l'amplitude de ladite première impulsion n'est pas supérieure à une valeur de référence, qui est plus petite qu'une amplitude d'au moins une deuxième impulsion desdites impulsions et ce, selon une proportion prédéterminée de l'amplitude de ladite deuxième impulsion, l'amplitude de ladite deuxième impulsion étant déterminée par ledit moyen de détermination d'amplitudes d'impulsions après que l'amplitude de ladite première impulsion a été déterminée ; et
un moyen de détermination de la pression sanguine (58) servant à déterminer, en tant que valeur de la pression sanguine diastolique de contrôle (MBP_{DIA}), ladite pression dudit brassard correspondant à ladite amplitude de ladite première impulsion, lorsque ledit moyen de détermination de valeurs candidates détermine, en tant que ladite valeur candidate de la pression sanguine diastolique, ladite pression dudit brassard correspondant à ladite amplitude de ladite première impulsion, par rapport à un nombre prédéterminé de ladite au moins une deuxième impulsion.

2. Dispositif de surveillance de la pression sanguine selon la revendication 1, dans lequel ledit moyen de détermination de valeurs candidates (56) comprend un moyen d'estimation (S6-6) afin d'établir si l'amplitude de ladite première impulsion n'est pas supérieure à une valeur de référence, qui est plus petite qu'une amplitude de chacune d'une pluralité de deuxièmes impulsions desdites impulsions et ce, selon une proportion prédéterminée de l'amplitude de ladite chaque deuxième impulsion, les amplitudes respectives desdites deuxièmes impulsions étant déterminées par ledit moyen de détermination d'amplitudes d'impulsions après que l'amplitude de ladite première impulsion a été déterminée.

3. Dispositif de surveillance de la pression sanguine selon la revendication 1 ou la revendication 2, dans lequel ledit dispositif de réglage de pression de brassard (52) comprend un moyen d'augmentation de pression (S6-2) servant à une augmentation pas à pas de la pression dudit brassard, en procédant, en alternance, à une augmentation de la pression du brassard et au maintien de la pression du brassard à chacune d'une pluralité de valeurs différentes de la pression, et dans lequel ledit moyen de détermination d'amplitudes d'impulsions (54) détermine une amplitude d'au moins une impulsion, qui est produite dans ledit brassard et détectée par ledit capteur de pression alors que la pression du brassard est maintenue à ladite chaque valeur de la pression.

4. Dispositif de surveillance de la pression sanguine selon l'une quelconque des revendications 1 à 3, dans lequel ledit moyen de détermination de la pression sanguine comprend un moyen de surveillance (S5) servant à la détermination itérative de ladite valeur de la pression sanguine diastolique de contrôle.

5. Dispositif de surveillance de la pression sanguine selon la revendication 4, comprenant en outre un moyen d'identification d'anomalies (60) afin d'identifier une anomalie des valeurs de la pression sanguine diastolique de contrôle, déterminées de manière itérative par ledit moyen de surveillance.

6. Dispositif de surveillance de la pression sanguine selon la revendication 5, dans lequel ledit moyen d'identification d'anomalies comprend un moyen (S8) servant à identifier ladite anomalie sur la base d'au moins l'une d'une grandeur de modification d'une valeur, déterminée en dernier lieu, desdites valeurs de la pression sanguine diastolique de contrôle à partir d'une moyenne desdites valeurs de la pression sanguine diastolique de contrôle, et d'une vitesse de modification de ladite valeur, déterminée en dernier lieu, desdites valeurs de la pression sanguine diastolique de contrôle à partir de ladite moyenne desdites valeurs de la pression sanguine diastolique de contrôle.

7. Dispositif de surveillance de la pression sanguine selon la revendication 5 ou la revendication 6, comprenant, en outre, un dispositif de mesure de la pression sanguine (10, 12, S3), qui augmente la pression exercée par ledit brassard jusqu'à une pression cible, qui est plus élevée qu'une pression sanguine systolique du sujet, et qui mesure au moins l'une d'une valeur de la pression sanguine systolique, de la pression sanguine moyenne et de la pression sanguine diastolique de l'être vivant, sur la base d'une variation d'amplitudes respectives d'impulsions d'une onde impulsionnelle, qui sont produites dans ledit brassard et détectées par ledit capteur de pression pendant au moins l'une d'une augmentation de la pression exercée par le brassard jusqu'à ladite pression cible, et d'une diminution de la pression exercée par le brassard à partir de ladite pression cible.

8. Dispositif de surveillance de la pression sanguine selon la revendication 7, dans lequel ledit dispositif de mesure de la pression sanguine comprend un moyen (S9) servant à mesurer ladite au moins l'une de ladite valeur de la pression sanguine systolique, de la pression sanguine moyenne et de la pression sanguine diastolique de l'être vivant lorsque ledit moyen d'identification d'anomalies identifie ladite anomalie.

9. Dispositif de surveillance de la pression sanguine selon l'une quelconque des revendications 1 à 8, comprenant, en outre, un dispositif afficheur (38) qui présente ladite valeur de la pression sanguine diastolique de contrôle, déterminée par ledit moyen de détermination de la pression sanguine.

10. Dispositif de surveillance de la pression sanguine selon l'une quelconque des revendications 1 à 9, dans lequel ladite proportion prédéterminée est de 0,3.

11. Dispositif de surveillance de la pression sanguine selon l'une quelconque des revendications 1 à 10, dans lequel ledit nombre prédéterminé est de trois.
